# EUROPEAN PATENT APPLICATION

(11) **EP 3 605 406 A1**
(43) Date of publication of application: **05.02.2020**
(21) Application number: 18775495.7
(22) Date of filing: 28.03.2018
(51) Int. Cl.: G06N 99/00, G01N 33/483

(54) **LEARNING RESULT OUTPUT APPARATUS AND LEARNING RESULT OUTPUT PROGRAM**

(30) Priority: 29.03.2017 JP 2017064387
(71) Applicant: ThinkCyte, Inc., Tokyo 113-8485 (JP); The University of Tokyo, Bunkyo-ku, Tokyo 113-8654 (JP)
(72) Inventor: KAMESAWA Ryosuke, Tokyo 113-8485 (JP); OTA Sadao, Tokyo 113-8654 (JP)
(74) Representative: Zimmermann, Tankred Klaus
(86) International application number: PCT/JP2018/012708
(87) International publication number: WO 2018/181458

(57) **Abstract**

A learning result output apparatus includes a machine learning unit that performs machine learning on at least one of attributes of a learning target, with a degree of the attribute as an evaluation axis, on the basis of morphological information indicating a shape of the learning target, and a graph information generation unit that generates graph information indicating a graph representing a learning result obtained by performing machine learning in the machine learning unit, with the evaluation axis as an axis, on the basis of a learning model indicating the learning result.

## Description

### [Technical Field]

The present invention relates to a learning result output apparatus and a learning result output program.

Priority is claimed on Japanese Patent Application No. 2017-064387, filed March 29, 2017, the content of which is incorporated herein by reference.

### [Background Art]

In the related art, a flow cytometry method in which a measurement target is fluorescently stained and features of the measurement target are evaluated using a total amount of fluorescent light luminance, or a flow cytometer using this flow cytometry method is known (for example, Patent Literature 1). Further, a fluorescence microscope or an imaging cytometer that evaluates particulates such as cells or bacteria that are a measurement target using an image is known. In addition, an imaging flow cytometer that captures morphological information of particulates at high speed with the same throughput as a flow cytometer is known (for example, Patent Literature 2).

### [Citation List]

### [Patent Literature]

[Patent Literature 1] Japanese Patent No. 5534214
[Patent Literature 2] US Patent No. 6249341

### [Summary of Invention]

### [Technical Problem]

In the conventional art, the feature of the measurement target is indicated by a predetermined evaluation axis such as a total amount of fluorescent luminance or scattered light. The predetermined evaluation axis is determined by a measurer measuring the measurement target. However, the feature of the measurement target is not limited to the total amount of fluorescence or scattered light. A feature that cannot be represented in a graph used in the conventional art (e.g. a histogram or a scatter plot) or that has not been noticed by the measurer is also included in the feature of the measurement target. A two-dimensional spatial feature such as morphological information of cells or molecular localization is one of the examples of this type of feature. Since this feature includes a feature that cannot be displayed by a previously existing graph display method or a feature that the measurer has not noticed, there is a problem in that the feature of the measurement target cannot be represented with the predetermined evaluation axis or graph display method of the related art, and a particle group of the measurement target having such features cannot be selectively visualized (gated) and separated (sorted).

An object of the present invention is to provide a learning result output apparatus and a learning result output program that classify particle groups on the basis of morphological information of a measurement target.

### [Solution to Problem]

An aspect of the present invention is a learning result output apparatus, including: a machine learning unit that performs machine learning on at least one of attributes of a learning target, using the degree of an attribute as an evaluation axis, on the basis of morphological information indicating a shape of the learning target; and a graph information generation unit that generates graph information indicating a graph representing achieved results of machine learning by the machine learning unit, using above described axis as an evaluation axis, on the basis of a learning model indicating the learning result.

Further, according to an aspect of the present invention, the learning result output apparatus further includes an operation detection unit that detects an operation of selecting the evaluation axis based on the learning model, wherein the graph information generation unit generates the graph information using the evaluation axis selected by the operation detected by the operation detection unit as an axis.

Further, according to an aspect of the present invention, in the learning result output apparatus, the operation detection unit further detects a visualization operation of the learning target based on the graph information generated by the graph information generation unit.

Further, according to an aspect of the present invention, the learning result output apparatus further includes a control signal generation unit that generates a control signal that is used for distribution of the learning target on the basis of the visualization operation detected by the operation detection unit.

Further, according to an aspect of the present invention, in the learning result output apparatus, the morphological information is a time-series signal of an optical signal indicating the learning target detected by one or a few pixel detection elements while changing a relative position between the learning target and any one of an optical system having a structured lighting pattern and a structured detection system having a plurality of regions having different optical characteristics, using any one or both of the optical system and the detection system.

Further, an aspect of the present invention is a learning result output program for causing a computer to execute: a machine learning step of performing machine learning on at least one of attributes of the learning target, using the degree of a attribute as an evaluation axis, on the basis of morphological information indicating a shape of the learning target; and a graph information generation step of generating graph information indicating a graph representing learning result obtained by performing machine learning in the machine learning step, using the evaluation axis as an axis, on the basis of a learning model indicating the learning result.

### [Advantageous Effects of Invention]

According to the present invention, it is possible to provide a learning result output apparatus and a learning result output program that classify particle assemblages on the basis of the morphological information of the measurement target.

### [Brief Description of Drawings]

Fig. 1 is a diagram illustrating an appearance configuration of a cell measurement system.
Fig. 2 is a diagram illustrating an example of a functional configuration of a learning result output apparatus.
Fig. 3 is a diagram illustrating an example of a determination result obtained by determining certain signal information a machine learning unit.
Fig. 4 is a diagram illustrating an example of graph information generated by a display data generation unit.
Fig. 5 is a diagram illustrating an example of a graph displayed by a previously existing flow cytometer and graph information generated by the display data generation unit in the present invention.
Fig. 6 is a diagram illustrating an example of the graph information generated by the display data generation unit.
Fig. 7 is a flowchart illustrating an example of an operation of the learning result output apparatus.
Fig. 8 illustrates an example of a graph in which two axes are evaluation axe based on learning results.

### [Description of Embodiments]

### [Embodiment]

Hereinafter, an embodiment of a learning result output apparatus will be described with reference to the drawings.

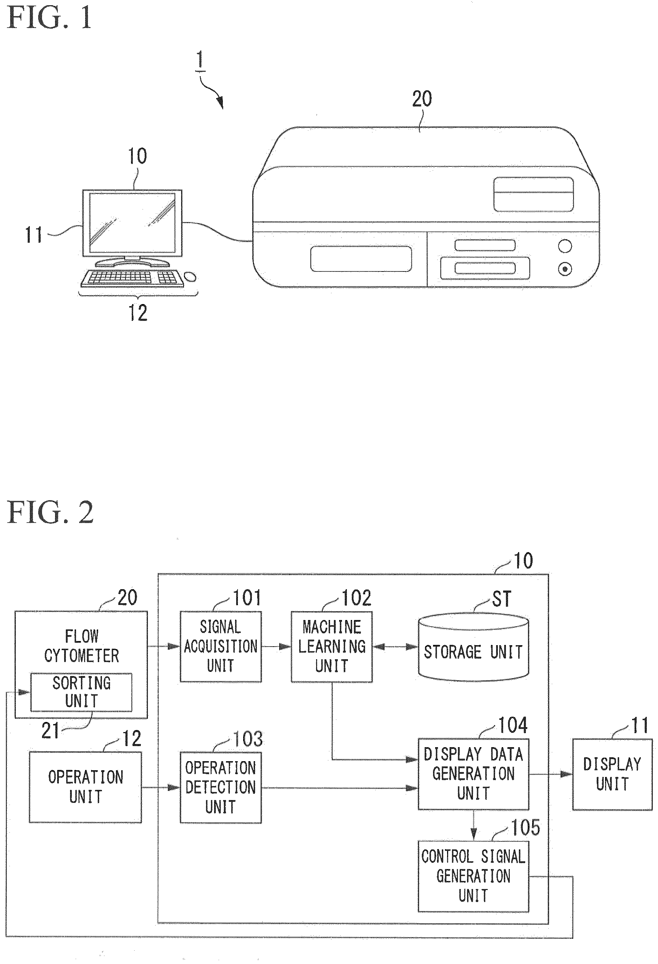

Fig. 1 is a diagram illustrating an appearance configuration of a cell measurement system 1.

The cell measurement system 1 includes a flow cytometer 20, a learning result output apparatus 10, a display unit 11, and an operation unit 12. The learning result output apparatus 10 performs machine learning on a signal including information of a measurement target measured by the flow cytometer 20. The learning result output apparatus 10 analyzes a feature of the measurement target through this machine learning.

### [Flow cytometer]

The flow cytometer 20 detects an optical signal of the measurement target such as a cell. The measurement target is an example of a learning target. Specifically, the measurement target is a cell. In the following description, the measurement targets are also described as particuleate assemblages. The flow cytometer 20 includes a flow path (not illustrated). The flow cytometer 20 generates a time-series signal of the optical signal from the measurement target flowing through this flow path.

### [Optical signal]

The optical signal is a time-series signal of an optical signal indicating the measurement target detected by one or a few pixel detection elements while changing a relative position between the measurement target and any one of an optical system having a structured lighting pattern and a structured detection system having a plurality of regions having different optical characteristics, using any one or both of the optical system and the detection system.

Specifically, the optical signal is information indicating an intensity of light detected by a sensor (not illustrated) included in the flow cytometer 20. The sensor is an example of one or a few pixel detection elements. One or a few pixel detection elements, specifically, are, for example, a single light reception element or a few light reception elements such as a photomultiplier tube (PMT), a line type PMT element, an avalanche photodiode (APD), or a photo-detector (PD), a CCD camera, and a CMOS sensor. The light detected by the sensor is the light modulated with the measurement target and an optical spatial modulator (not illustrated) from an irradiation unit (not illustrated) included in the flow cytometer 20. Here, the optical spatial modulator is an example of the structured lighting pattern.

The flow cytometer 20 detects the optical signal using one or a few pixel detection elements while changing the relative position between the measurement target and any one of the optical system and the detection system. In this example, the relative position between the optical system and the detection system is changed when the measurement target flows through the flow path.

### [Optical system and detection system]

The optical system will be describedherein. When the optical system includes an illumination unit and an optical spatial modulator, the detection system includes the sensor described above. This configuration is also described as a structured lighting configuration.

When the optical system includes an irradiation unit, the detection system includes an optical spatial modulator and a sensor. This configuration is also described as a structured detection configuration.

The flow cytometer 20 may have either the structured lighting configuration or the structured detection configuration.

### [Time series signal of optical signal]

The time-series signal of the optical signal is a signal in which times when a plurality of optical signals have been acquired and information on light intensities are associated with each other.

The flow cytometer 20 can reconstruct an image of the measurement target from this time-series signal. The time-series signal includes information on attributes of the measurement target. Specifically, the attributes include a shape of the measurement target, components constituting the measurement target, and the like. When the measurement target is fluorescently stained, information such as a degree of luminance of fluorescence from the measurement target is included. It should be noted that the learning result output apparatus 10 analyzes a feature of the measurement target without reconstructing the image of the measurement target.

### [Learning result output apparatus 10]

The learning result output apparatus 10 acquires the time-series signal of the optical signal detected by the flow cytometer 20. The learning result output apparatus 10 performs machine learning on the time-series signal acquired from the flow cytometer 20. The learning result output apparatus 10 analyzes the attributes of the measurement target through this machine learning.

The display unit 11 displays an analysis result of the learning result output apparatus 10.

The operation unit 12 receives an input from an operator operating the learning result output apparatus 10. Specifically, the operation unit 12 is a keyboard, a mouse, a touch panel, or the like.

A functional configuration of the learning result output apparatus 10 will be described herein with reference to Fig. 2.

Fig. 2 is a diagram illustrating an example of a functional configuration of the learning result output apparatus 10.

The learning result output apparatus 10 includes a signal acquisition unit 101, a machine learning unit 102, a storage unit ST, an operation detection unit 103, a display data generation unit 104, a display unit 11, and a control signal generation unit 105. Here, the display data generation unit 104 is an example of a graph information generation unit.

The signal acquisition unit 101 acquires signal information indicating the time-series signal from the flow cytometer 20 described above. Here, the signal information is an example of morphological information indicating the shape of the learning target. The signal acquisition unit 101 supplies the signal information acquired from the flow cytometer 20 to the machine learning unit 102.

The machine learning unit 102 performs machine learning on at least one of the attributes of the learning target, using the degree of this attribute as an evaluation axis. Specifically, the machine learning unit 102 acquires the signal information from the signal acquisition unit 101. The machine learning unit 102 forms a determiner by performing machine learning on the signal information acquired from the signal acquisition unit 101. Here, in the machine learning unit 102, the determiner is formed using a machine learning algorithm such as a support vector machine. This determiner is configured of a logic circuit of a field-programmable gate array (FPGA). It should be noted that the determiner may be configured of a programmable logic device (PLD), an application-specific integrated circuit (ASIC), or the like. The determiner is an example of a learning model.

Further, in the embodiment, in the machine learning unit 102, the determiner has been formed through machine learning with a teacher in advance.

The machine learning unit 102 determines the acquired signal information using the determiner.

The machine learning unit 102 supplies the determination result of determining the signal information to the display data generation unit 104. The determination result includes, for at least one of the attributes of the measurement target, information in which a degree of the attribute is used as the evaluation axis.

The operation detection unit 103 detects an operation of selecting the evaluation axis based on a determination result of the determiner. Specifically, the operation detection unit 103 detects an operation in which the operator selects an evaluation axis from among plurality of evaluation axes relating to the degrees of attributes. The operation detection unit 103 supplies information indicating the evaluation axis selected by the operator to the display data generation unit 104 on the basis of the detected operation. Additionally, the operation detection unit 103 further detects a visualization operation of the measurement target based on graph information generated by the display data generation unit 104. Specifically, the operation detection unit 103 detects an operation in which a user gates the measurement target on the basis of the graph information generated by the display data generation unit 104 to be described below. The gating will be described below.

The display data generation unit 104 generates graph information indicating a graph representing the determination result using the evaluation axis as an axis, on the basis of a determination result obtained by the machine learning unit 102 determining the signal information using the determiner. Specifically, the display data generation unit 104 acquires the determination result from the machine learning unit 102. The display data generation unit 104 acquires the information indicating the evaluation axis selected by the operator from the operation detection unit 103.

### [Determination result]

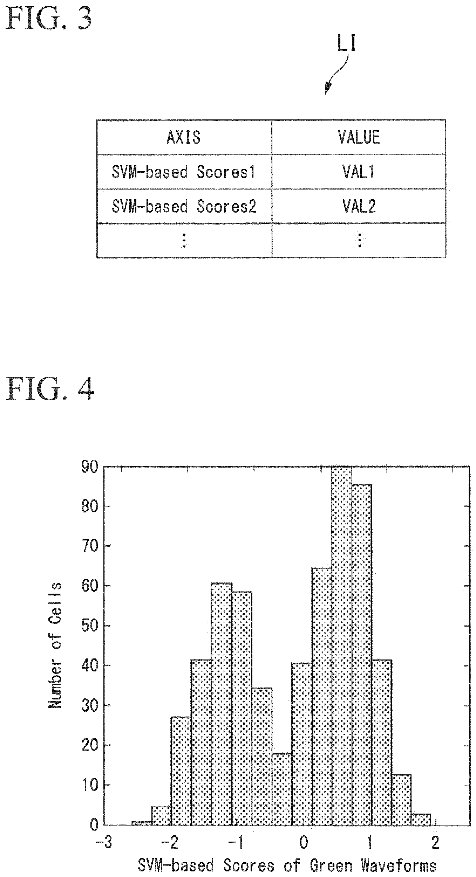

A determination result LI will be described herein with reference to Fig. 3.

Fig. 3 is a diagram illustrating an example of the determination result made by the machine learning unit 102, and the machine learning unit 102 makes it from certain signal information.

The determination result LI is information in which an evaluation axis indicating an attribute of a measurement target is associated with a value indicating the degree of an attribute. Specifically, the determination result LI includes "SVM-based Scores 1" as information on the evaluation axis and "VAL 1" as a value indicating the degree of the attribute in an associated state. Further, the determination result LI includes "SVM-based Scores 2" as information of the evaluation axis and "VAL 2" as a value indicating the degree of the attribute in an associated state.

Returning to Fig. 2, the display data generation unit 104 generates graph information of which the evaluation axis selected by the operator is an axis. The graph information is information indicating a graph representing the determination result of the measurement target. Specifically, the graph information is information including information in which at least one axis of the determination result LI is the evaluation axis .

The display data generation unit 104 supplies the generated graph information to the display unit 11. The display unit 11 displays the graph information as a displayed image.

The display data generation unit 104 acquires a gating operation indicating the operation gated by a user from the operation detection unit 103. The display data generation unit 104 supplies information indicating the measurement target selected by this gating operation to the control signal generation unit 105. In the following description, a measurement target selected by the gating operation will also be described as a selected measurement target. Specifically, the selected measurement target is determined by gating a measurement target of interest to the user who operates the learning result output apparatus 10. In the following description, gating is also described as selective visualization. Through this gating, the learning result output apparatus 10 can perform analysis on target cells things by removal of dusts or particles other than target cells contained in the measurement target.

More specifically, sorting is that the flow cytometer 20 distributes a particulate group gated by the user who operates the learning result output apparatus 10.

The gating is performed by the user who operates the learning result output apparatus 10. The user performs a gating operation on the basis of the graph information generated by the display data generation unit 104. The operation detection unit 103 detects this user operation.

The control signal generation unit 105 generates a control signal that is used for distribution of the learning target on the basis of the visualization operation. The control signal generation unit 105 acquires information indicating the selected measurement target from the display data generation unit 104, The control signal generation unit 105 generates a control signal that is used for sorting, on the basis of the information indicating the selected measurement target acquired from the display data generation unit 104. Sorting is selective separation of the measurement target. The separation is, in this example, selective separating according to the evaluation axis. The sorting is an example of the distribution. The control signal is a signal for controlling the sorting unit 21 included in the flow cytometer 20. The control signal generation unit 105 supplies the generated control signal to the sorting unit 21.

The sorting unit 21 acquires the control signal from the control signal generation unit 105. The sorting unit 21 sorts the selected measurement target among the measurement targets flowing through the flow path on the basis of the control signal acquired from the control signal generation unit 105.

### [Graph information]

The graph information generated by the display data generation unit 104 will be herein with reference to Figs. 4 to 6.

Fig. 4 is a diagram illustrating an example of the graph information generated by the display data generation unit 104.

The graph illustrated in Fig. 4 is a graph generated on the basis of the determination result LI. This graph shows the number of corresponding measurement targets to each degree of the attribute shown on an evaluation axis.

A horizontal axis of the graph illustrated in Fig. 4 is an evaluation axis "SVM-based Scores of Green Waveforms". As described above, this evaluation axis is an axis included in the determination result LI that is a result of machine learning by the machine learning unit 102. A vertical axis of this graph is the number of measurement targets.

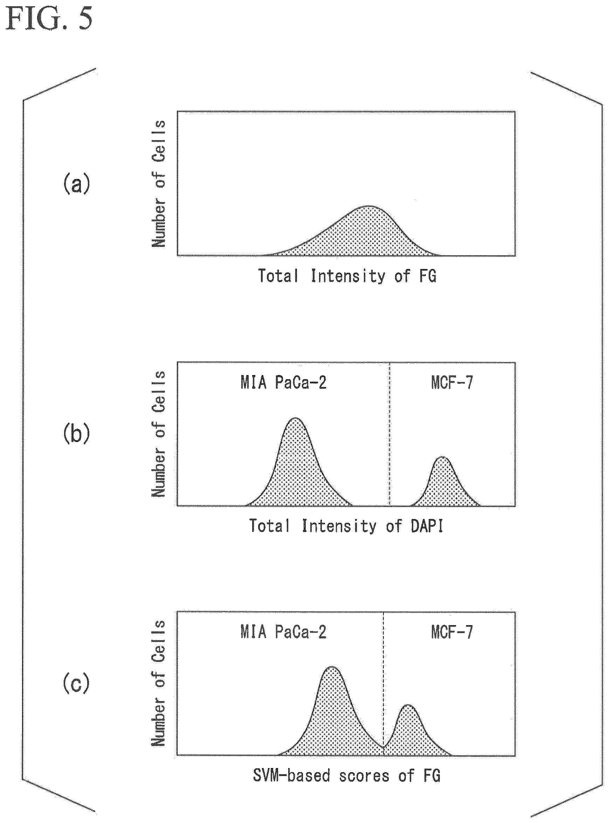

Fig. 5 is a diagram illustrating an example of a graph displayed by a conventional flow cytometer and the graph information generated by the display data generation unit 104. A measurement target illustrated in Fig. 5 is a plurality of cells fluorescently stained with DAPI (4',6-diamidino-2-phenylindole) and FG (fixable green). The machine learning unit 102 performs machine learning on signal information for each cell. The DAPI is a staining agent for blue fluorescence. FG is a staining agent for green fluorescence.

Fig. 5(a) is the graph generated by a conventional flow cytometer. A horizontal axis in Fig. 5(a) indicates "Total Intensity of FG" that is a predetermined axis. A vertical axis in Fig. 5(a) indicates the number of measurement targets.

Fig. 5(b) is a graph generated by the display data generation unit 104 in the embodiment. A horizontal axis in Fig. 5(b) indicates "Total Intensity of DAPI" that is the evaluation axis included in the determination result LI. The evaluation axis "Total Intensity of DAPI" is an evaluation axis of the degree of intensity of blue fluorescence arising from the DAPI of two types of cell. A vertical axis in Fig. 5(b) is the number of measurement targets. Here, "MIA PaCa-2" and "MCF-7" shown in this graph are the above-described measurement targets. The machine learning unit 102 generates the determination result LI including the degree of the intensity of the blue fluorescence arising from the two types of cell. The display data generation unit 104 generates a graph including the degree of the intensity of the blue fluorescence of the two types of cell.

Fig. 5(c) is a graph generated by the display data generation unit 104 in the embodiment. A horizontal axis in Fig. 5(c) indicates "SVM-based scores of FG" that is the evaluation axis included in the determination result LI. This evaluation axis "SVM-based scores of FG" is an evaluation axis in which a score based on morphological information of the cells stained with the FG determined by the determiner is used as an axis. A vertical axis in Fig. 5(c) indicates the number of measurement targets. By using the "SVM-based scores of FG" including the morphological information of the measurement target as an axis, it becomes possible to represent two peaks "MIA PaCa-2" and "MCF-7", which could not be represented in a conventional histogram of a total amount of fluorescence of FG.

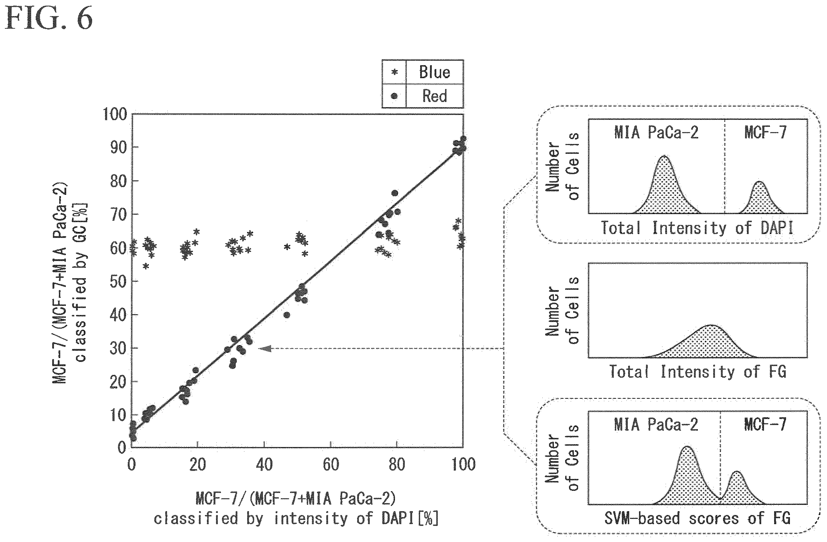

Fig. 6 is a diagram illustrating an example of the graph information generated by the display data generation unit 104.

A dot PT1 in the graph illustrated in Fig. 6 indicates the determination result LI illustrated in Figs. 5(b) and 5(c) described above. This graph illustrates a ratio of the number of a plurality of measurement targets. A horizontal axis of this graph indicates a ratio of "MCF-7" included in 600 cells, in which only the "MCF-7" in the 600 cells is stained with DAPI.

In a vertical axis of this graph, an entire cell cytoplasm of "MCF-7" and "MIA PaCa-2" in the 600 cells is stained with FG. Blue dots show cases in which the ratio of "MCF-7" included in the 600 cells has been discriminated on the basis of a total amount of fluorescence of FG, and red dots indicate a ratio of "MCF-7" which is judged by machine learning on the basis of morphological information of the cytoplasm stained with FG that "MCF-7" is included. That is, the blue dots are obtained by plotting the results of discrimination based on correct data on the horizontal axis and the results based on morphological information of the cells on the vertical axis. Thus, this shows that the learning result output apparatus 10 can discriminate a cell group more accurately, which could not be correctly discriminated by a conventional approach where the cell group is discriminated using only total amount of fluorescence as indicated with blue dots, by using machine learning for cells morphologies as indicated with red dots.

### [Overview of operation of learning result output apparatus 10]

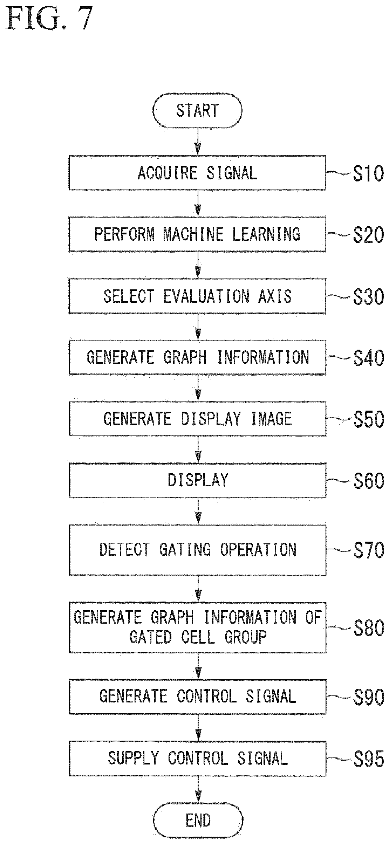

Next, an overview of the operation of the learning result output apparatus 10 will be described with reference to Fig. 7.

Fig. 7 is a flowchart illustrating an example of the operation of the learning result output apparatus 10.

The signal acquisition unit 101 acquires the signal information from the flow cytometer 20 (step S10). The signal acquisition unit 101 supplies the signal information acquired from the flow cytometer 20 to the machine learning unit 102.

The machine learning unit 102 acquires the signal information from the signal acquisition unit 101. The machine learning unit 102 performs machine learning on the signal information acquired from the signal acquisition unit 101 (step S20). The machine learning unit 102 supplies the determination result LI that is a result of machine learning to the display data generation unit 104. The machine learning unit 102 supplies the determination result LI to the control signal generation unit 105.

The display data generation unit 104 acquires the determination result LI from the machine learning unit 102. The display data generation unit 104 causes the display unit 11 to display the determination result LI acquired from the machine learning unit 102. The operator selects the evaluation axis included in the determination result LI displayed on the display unit 11 (step S30). The operation detection unit 103 detects this operation by the operator. The operation detection unit 103 supplies the information indicating the evaluation axis selected by the operator to the display data generation unit 104.

The display data generation unit 104 acquires the information indicating the evaluation axis selected by the operator from the operation detection unit 103. The display data generation unit 104 generates graph information in which the axis selected by the operator, which has been acquired from the operation detection unit 103, is the evaluation axis (step S40). The display data generation unit 104 supplies the generated graph information to the display unit 11.

The display unit 11 acquires the graph information from the display data generation unit 104. The display unit 11 generates a displayed image on the basis of the graph information (step S50). The display unit 11 displays the generated image on screen (step S60).

The user operating the learning result output apparatus 10 performs gating on the basis of the displayed image. The operation detection unit 103 detects this gating operation as a gating operation (step S70). The operation detection unit 103 supplies the detected gating operation to the display data generation unit 104. The display data generation unit 104 acquires the gating operation from the operation detection unit 103. The display data generation unit 104 generates graph information of the gated cell group on the basis of the gating operation acquired from the operation detection unit 103 (step S80).

The display data generation unit 104 supplies selected measurement target information indicating the selected measurement target selected by the gating operation to the control signal generation unit 105. The control signal generation unit 105 acquires the selected measurement target information from the display data generation unit 104. The control signal generation unit 105 generates a control signal indicating a signal that is used for sorting of the selected measurement target on the basis of the selected measurement target information acquired from the display data generation unit 104 (step S90).

The control signal generation unit 105 supplies the generated control signal to the sorting unit 21 (step S95).

The sorting unit 21 acquires the control signal from the control signal generation unit 105. The sorting unit 21 sorts the selected measurement targets from among the measurement targets flowing through the flow path on the basis of the control signal.

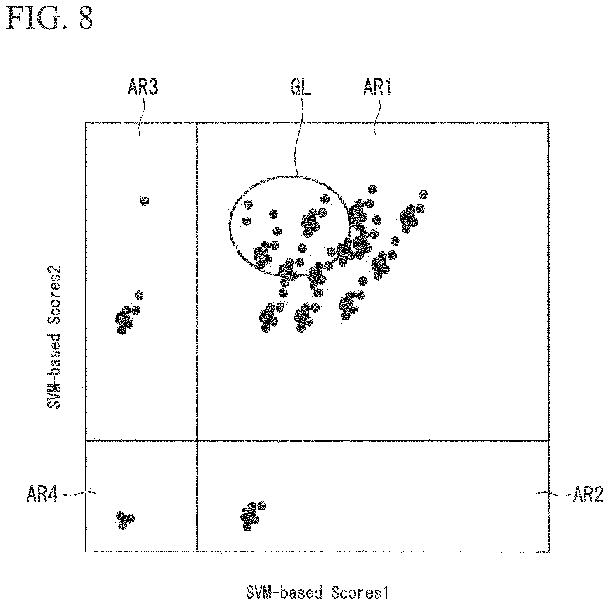

An example of the gating operation detected by the operation detection unit 103 will be describedherein with reference to Fig. 8.

Fig. 8 is an example of a graph in which two axes are evaluation axes based on the determination result LI.

The graph illustrated in Fig. 8 shows a determination result of the measurement signal in which a horizontal axis is "SVM-based Scores 1" and a vertical axis is "SVM-based Scores 2".

Dots included in an area AR1 are the dots which show measurement targets having both an attribute indicated by "SVM-based Scores 1" and an attribute indicated by "SVM-based Scores 2". Dots included in the area AR2 are the dots which show measurement targets having only the attribute indicated by "SVM-based Scores 1". Dots included in the area AR3 are the dots which show measurement targets having only the attribute indicated by "SVM-based Scores 2". Dots included in the area AR4 are the dots which show measurement targets having neither the attribute indicated by "SVM-based Scores 1" nor the attribute indicated by "SVM-based Scores 2".

The user operating the learning result output apparatus 10 selects an area thought to include dots of a target cell group from among points indicating measurement targets, and sets a boundary GL. Setting the boundary GL is gating. It should be noted that the user presumes a strength of a total amount of scattered light or fluorescence, and morphological information from past data or the like, and configure an area which is thought to enclose the target cell group to set the boundary.

The operation detection unit 103 detects this gating operation. The operation detection unit 103 supplies the detected gating operation to the display data generation unit 104. The display data generation unit 104 draws the boundary GL on the basis of the gating operation.

Further, the display data generation unit 104 may generate graph information of the cell group included in the boundary GL. The graph information of the cell group included in the boundary GL is, for example, a graph such as a histogram or a scatter plot illustrated in Figs. 5 and 6 described above.

### [Conclusion]

As described above, the learning result output apparatus 10 includes the signal acquisition unit 101, the machine learning unit 102, and the display data generation unit 104. The signal acquisition unit 101 acquires the signal information from the flow cytometer 20. This signal information includes various pieces of information of the measurement target. The machine learning unit 102 performs the determination on the basis of the signal information. The machine learning unit 102 generates the determination result LI. The determination result LI generated by the machine learning unit 102 includes the evaluation axis that is the attribute of the measurement target. The display data generation unit 104 generates the graph information indicating the determination result LI with the evaluation axis of the degree of the attribute as an axis, on the basis of the determination result LI machine-learned by the machine learning unit 102. Accordingly, the learning result output apparatus 10 can generate a graph having the evaluation axis included in the determination result LI as an axis. Further, the learning result output apparatus 10 can generate a graph in which the evaluation axes included in the determination result LI are combined. Accordingly, the learning result output apparatus 10 can generate information using the degrees of various attributes of the measurement target as axes. On the basis of this information, the learning result output apparatus 10 can classify particle groups on the basis of the morphological information of the measurement target.

It should be noted that although the configuration in which the signal acquisition unit 101 acquires the signal information from the flow cytometer 20 has been described above, the present invention is not limited thereto. The signal acquisition unit 101 may acquire the signal information from another device.

It should be noted that although the configuration in which the learning result output apparatus 10 includes the operation detection unit 103 has been described above, this is not essential. The learning result output apparatus 10 may generate the graph information representing a machine learning result with the evaluation axis as an axis. The learning result output apparatus 10 can detect the selection of the operator by including the operation detection unit 103. The operator operating the learning result output apparatus 10 can recognize a feature that the operator has not noticed, by selecting the evaluation axis included in the determination result LI. Further, since the learning result output apparatus 10 can generate a graph based on a feature that the operator has not noticed, it is possible to analyze the measurement target in more detail.

Further, the learning result output apparatus 10 classifies, feature quantities regarding the morphological information of the cells, which cannot be made by the conventional art. Accordingly, the learning result output apparatus 10 can display a feature quantity of a measurement target, which cannot be made by the conventional art.

Further, the learning result output apparatus 10 can detect the above-described gating operation by including the operation detection unit 103.

The learning result output apparatus 10 includes the control signal generation unit 105. The control signal generation unit 105 generates a control signal on the basis of the gating operation detected by the operation detection unit 103. The cell group selected by this gating operation is based on the graph with the evaluation axis based on the learning result LI. When this evaluation axis is the evaluation axis of the morphological information indicating the morphologies of the cells, the user can gate the target cells on the basis of the morphologies of the cells. The flow cytometer 20 can sort the target cells on the basis of the control signal generated by the control signal generation unit 105.

That is, the learning result output apparatus 10 can detect the gating operation based not only on the intensity of the scattered light or the fluorescence from the cell group in the conventional art, but also on a graph with the evaluation axis included in the learning result LI as an axis. Further, the learning result output apparatus 10 can generate a control signal for separating the selected cell group by detecting this gating operation.

Further, the machine learning unit 102 includes a determiner configured of a logic circuit. Accordingly, the machine learning unit 102 can achieve machine learning on the measurement target in a short time. That is, the learning result output apparatus 10 can generate the determination result LI including various attributes of the measurement target in a short time.

It should be noted that although the configuration in which the machine learning unit 102 performs the machine learning using a support vector machine has been described above, the present invention is not limited thereto. The machine learning unit 102 may be configured to supply the degree of the attribute of the measurement target as the machine learning result to the display data generation unit 104. For example, a configuration in which the machine learning unit 102 performs machine learning using a random forest, a neural network, or the like may be adopted. Further, the machine learning unit 102 may have no teacher as long as the machine learning unit is a machine learning model that outputs an attribute regarding a target. Examples of the machine learning model that outputs an attribute regarding a target may include principal component analysis, auto encoder, or the like.

It should be noted that, although the configuration in which the learning result output apparatus 10 includes the control signal generation unit 105 has been described above, the control signal generation unit 105 is not essential. By including the control signal generation unit 105, the learning result output apparatus 10 can perform control of sorting on the flow cytometer 20 on the basis of the evaluation axis included in the determination result LI.

It should be noted that although the configuration in which, in the flow cytometer 20 described above, a relative position of the measurement target is changed with respect to the optical system or the detection system has been described, the present invention is not limited thereto. The optical system or the detection system may be moved to a stationary measurement target.

Further, although the configuration in which the flow cytometer 20 described above acquires the time-sequential signal of the optical signal has been described, the present invention is not limited thereto. The flow cytometer 20 may be an imaging flow cytometer. In this case, the imaging flow cytometer is a flow cytometer that captures an image of a measurement target using an imaging device such as a charge-coupled device (CCD), a complementary MOS (CMOS), or a photomultiplier tube (PMT). The imaging flow cytometer generates a captured image indicating the captured image. The flow cytometer 20 supplies this captured image to the learning result output apparatus 10 as signal information. The learning result output apparatus 10 generates the determination result LI by determining the image of the measurement target included in the captured image using the determiner included in the machine learning unit 102.

It should be noted that although the representation of the graph illustrated in Fig. 8 described above is an example, the present invention is not limited thereto. The display data generation unit 104 may generate graph information in which each of the two axes is an evaluation axis based on the determination result LI.

Although the embodiment of the present invention has been described in detail with reference to the drawings, a specific configuration is not limited to this embodiment, and appropriate changes can be made without departing from the spirit of the present invention.

It should be noted that the above-described learning result output apparatus 10 has a computer therein. Steps of the respective processes of the above-described apparatus are stored in a format of a program in a computer-readable recording medium, and the various processes are performed by a computer reading and executing this program. Further, the computer-readable recording medium refers to a magnetic disk, a magneto-optical disk, a CD-ROM, a DVD-ROM, a semiconductor memory, or the like. Further, this computer program may be distributed to a computer through a communication line, and the computer that has received this distribution may execute the program.

Further, the program may be a program for realizing some of the above-described functions.

Further, the program may be a so-called difference file (difference program) that can realize the above-described functions in combination with a program already recorded in a computer system.

### [Reference Signs List]

- 1: Cell measurement system
- 10: Learning result output apparatus
- 20: Flow cytometer
- 21: Sorting unit
- 11: Display unit
- 12: Operation unit
- 101: Signal acquisition unit
- 102: Machine learning unit
- 103: Operation detection unit
- 104:: Display data generation unit
- 105: Control signal generation unit

## Claims

1. A learning result output apparatus, comprising:
a machine learning unit that performs machine learning on at least one of attributes of a learning target, using the degree of an attribute as an evaluation axis, on the basis of morphological information indicating shape of the learning target; and
a graph information generation unit that generates graph information indicating a graph representing a learning result obtained by performing machine learning in the machine learning unit, with the evaluation axis as an axis, on the basis of a learning model indicating the learning result.

2. The learning result output apparatus according to Claim 1, further comprising:
an operation detection unit that detects an operation of selecting the evaluation axis based on the learning model,
wherein the graph information generation unit generates the graph information using the evaluation axis selected by the operation detected by the operation detection unit as an axis.

3. The learning result output apparatus according to Claim 2, wherein the operation detection unit further detects a visualization operation of the learning target based on the graph information generated by the graph information generation unit.

4. The learning result output apparatus according to Claim 3, further comprising:
a control signal generation unit that generates a control signal that is used for sorting of the learning target on the basis of the visualization operation detected by the operation detection unit.

5. The learning result output apparatus according to any one of Claims 1 to 4, wherein the morphological information is a time-series signal of an optical signal indicating the learning target detected by one or a few pixel detection elements while changing a relative position between the learning target and any one of an optical system having a structured illumination pattern and a structured detection system having a plurality of regions having different optical characteristics, using any one or both of the optical system and the detection system.

6. A learning result output program for causing a computer to execute:
a machine learning step of performing machine learning on at least one of attributes of a learning target, with the degree of an attribute as an evaluation axis, on the basis of morphological information indicating a shape of the learning target; and
a graph information generation step of generating graph information indicating a graph representing a learning result obtained by performing machine learning in the machine learning step, with the evaluation axis as an axis, on the basis of a learning model indicating the learning result.
